# EUROPEAN PATENT APPLICATION

(11) **EP 1 347 045 A1**
(43) Date of publication of application: **24.09.2003**
(21) Application number: 01998628.0
(22) Date of filing: 30.11.2001
(51) Int. Cl.: C12N 9/58, C12N 15/57, C12N 15/63, A01H 5/00, A01N 63/00

(54) **ENZYME WITH PROTEOLYTIC ACTIVITY**

(30) Priority: 01.12.2000 ES 200002897
(71) Applicant: NEWBIOTECHNIC, S.A., 41092 Sevilla (ES); Universidad de Sevilla, 41013 Sevilla (ES); Universidad De Salamanca, E-37008 Salamanca (ES)
(72) Inventor: SUAREZ FERNANDEZ, Belén, 37008 Salamanca (ES); REY BARRERA, Manuel, 41092 Sevilla (ES); MONTE VAZQUEZ, Enrique, 37008 Salamanca (ES); LLOBELL GONZALEZ, Antonio, 41092 Sevilla (ES)
(86) International application number: ES0100471
(87) International publication number: WO02044359

(57) **Abstract**

This invention relates to enzymes with proteolytic activity, to DNA constructs that code for said enzymes, to methods for production of said enzymes, to compositions containing said enzymes and to the use of said enzymes and enzymatic preparations for the degradation or modification of materials that contain peptide bonds.

## Description

### FIELD OF THE INVENTION

This invention relates to enzymes with proteolytic activity, to DNA constructs that code for said enzymes, to methods for the production of said enzymes, to compositions comprising said enzymes and to the use of said enzymes, DNA constructs and compositions.

### BACKGROUND OF THE INVENTION

Enzymes with proteolytic activity, known as proteases, peptidases or peptide hydrolases, are able to catalyse the hydrolysis of the peptide bond (E.C. 3.4). This group of enzymes is characterised by its ubiquitous distribution in different life forms, its variability in the cell localisation and by its involvement in a large variety of physiological processes.

Depending on their mode of action, peptidases are classified as exopeptidases [peptidases that require the presence of an amino or free carboxyl terminal in the substrate], and endopeptidases [peptidases that hydrolyse peptide bonds present in the chain]. The different catalytic mechanisms recognised for these enzymes have given rise to four subgroups named according to the active site that intervenes directly in the rupture of the peptide bond [aspartyl-endopeptidases, cysteine-endopeptidases, metallo-endopeptidases and serine-endopeptidases]. A review of the classification and characteristics of these enzymes can be found in the work of Rawlings and Barret [Rawlings N.D. & Barrett A.J. (1994). Families of serine peptidases. Methods in Enzymology, 244:19-61; Rawlings N.D. & Barrett A.J. (1994). Families of cysteine peptidases. Methods in Enzymology, 244:461-486; Rawlings N.D. & Barrett A.J. (1995). Families of aspartic peptidases, and those of unknown catalytic mechanism. Methods in Enzymology, 248:105-120; Rawlings N.D. & Barrett A.J. (1995). Evolutionary families of metallopeptidases. Methods in Enzymology, 248:183-228). The peptidases can be acidic, basic or neutral, depending on their activity at low, high or neutral pH, respectively.

Peptidases are widely used in different industries, for example, in the manufacture of detergents for washing clothes, in the leather industry, in the food industry, etc.

Although the fungi of the genus *Trichoderma* have been under study for a long time (both because of their cellulolytic activity and because of their action as antagonists to fungal pathogens of plants) and numerous enzymes have been identified (chitinases, β-1,3- and β-1,6-glucanases, α-1,3-glucanases, etc.), only two proteases have been identified and characterised: an aspartyl-protease of *T.* reesei and a serine protease of *T. harzianum.* The latter one is denominated Prb1, has been related to mycoparasitism, has a molecular weight of 31 kDa, a basic isoelectric point (Ip) and belongs to the subtilisins family (Geremia, R.A., Goldman, G.H., Jacobs, D., Ardiles, W., Vila, S.B., van Motagu, M. and Herrera-Estrella, A. (1993). Molecular characterization of the proteinase-encoding gene, prb1, related to mycoparasitism by *Trichoderma harzianum.* Molecular Microbiology 83: 603-613).

Although numerous enzymes have been described with proteolytic activity, their importance in industry along with the enormous variety present in terms of mechanism of action, affinity for substrate, specificity, lytic capacity, etc., justify the need to extend the arsenal of proteolytic enzymes available.

### SUMMARY OF THE INVENTION

The invention addresses the problem of providing new enzymes with proteolytic activity.

The solution provided by this invention is based on a new enzyme discovered by the inventors which has proteolytic activity, an acid Ip and belongs to the family of serine peptidases. A characteristic that said enzyme presents, in addition to its proteolytic activity that allows it to degrade peptide bonds, is its affinity for structures that comprise proteins or peptides, such as cell walls of fungi (which contain chitin and glucan polymers embedded in, and covalently bound to, a protein matrix), or structures comprising polysaccharides covalently bound to proteins, for example, cuticles of insects, arachnids, etc., whereby said enzyme may intervene in the degradation or modification of said structures comprising proteins or peptides. In addition, the enzyme provided by the invention can also be used in the irreversible proteolytic deactivation of structural proteins or with enzymatic activity determining the virulence or pathogenesis of pathogens on animals or plants.

Therefore, an object of this invention constitutes an enzyme with proteolytic activity. The process for obtaining said enzyme and compositions comprising said enzyme also constitute additional objects of this invention.

Additional objects of this invention constitute the use of said enzymes or compositions for the degradation or modification of materials containing peptide bonds, including structures comprising proteins or peptides, as well as irreversible proteolytic deactivation of structural proteins or those with enzymatic activity determining the virulence or pathogenesis of pathogens on animals or plants.

Another additional object of this invention constitutes the isolation and characterisation of DNA sequences that encode said enzyme and the cloning of said DNA sequences. The vectors, cells and transgenic plants comprising said DNA sequences constitute another additional object of this invention. The use of said DNA sequences for obtaining said enzymes or for the construction of transgenic plants also constitutes an additional object of this invention.

### DETAILED DESCRIPTION OF THE INVENTION

The invention provides an enzyme with proteolytic activity, hereinafter the enzyme of the invention, which has a sequence of amino acids selected from:
a) a sequence of amino acids comprising the sequence of amino acids shown in SEQ. ID. No.: 1, and
b) a sequence of amino acids substantially homologous and functionally equivalent to the sequence of amino acids shown in SEQ. ID. NO.: 1.

In the sense used in this description, the expression "substantially homologous" means that the sequences of amino acids in question have a degree of identity, at the amino acid level, of at least 70%, preferably of at least 85%, and more preferably of at least 95%.

Similarly, in the sense used in this description, the expression "functionally equivalent" means that the protein in question has at least proteolytic activity.

The enzyme of the invention, in addition to its proteolytic activity, which can be assayed both in gel, by means of the casein-SDS-PAGE assay [Example 1.2] and in liquid phase [Section A of Example 3.3], can also have affinity for structures that comprise proteins or peptides, for example, the cell walls of fungi, the cuticles of insects and arachnids, etc., and/or the capacity to irreversible deactivate, by means of proteolysis, structural proteins or those with enzymatic activity implicated in the attack of a pathogen on animals or plants. The affinity of the enzyme of the invention for structures that comprise proteins or peptides can be assayed by means of an adsorption-digestion process with purified cell walls of a fungus, for example, C. acutatum or *Botrytis cinerea,* as described in Example 1.3, where an assay to determine the adsorption of proteins to fungal cell walls is illustrated.

In a particular embodiment, the enzyme of the invention has the sequence of amino acids shown in SEQ. ID. NO.: 1 or an active fragment thereof, that is, a fragment of said protein that maintains its proteolytic activity.

In another particular embodiment, the enzyme of the invention is an enzyme, or active fragment, derived from *T. harzianum*, which has the sequence of amino acids shown in SEQ. ID. NO.: 1, or a part thereof. In a specific embodiment, the enzyme of the invention is an enzyme derived from *T. harzianum* which has the sequence of amino acids shown in SEQ. ID. NO.: 1 and has been denominated Pral in this description [Examples 1 and 2].

The Pral enzyme has a molecular weight of 28 kDa as determined by SDS-PAGE, an Ip of 4.7-4.9, an optimum pH of 7.5 (although it is more stable at acid pH), an optimum temperature comprised between 35°C and 40°C (pH 7.5), belongs to the family of serine peptidases, is a trypsin type peptidase since it presents the sequences of the amino terminal and of an internal peptide presenting a high degree of homology with other proteases of the trypsin type, it has specificity for N-acetyl-Ile-Glu-Ala-Arg-pNA (a synthetic substrate specific for trypsin) but it does not have activity against other synthetic substrates for chymotrypsin or elastase [Example 3].

The enzyme of the invention can be obtained from a producing organism thereof, such as a fungus of the genus *Trichoderma,* by means of a process comprising the culture of the producing organism under conditions appropriate for the expression of said enzyme and, subsequently, recovering said enzyme. In a particular embodiment of this invention, the fungus used belongs to the species *Trichoderma harzianum*, specifically to the strain deposited in the Spanish Collection of Type Cultures (CECT) with the access number CECT 2413.

As mentioned in Example 1, the culture of producing organisms can be performed in two stages. In a first stage, the spores of the fungus are inoculated in a culture medium supplemented with glucose as a carbon source and, then, the mycelium is collected, washed and cultured in a suitable minimum medium supplemented with purified fungal cell walls, for example, of *Colletotrichum acutatum,* as the only carbon source for inducing enzymes with proteolytic activity.

The isolation and purification of the enzyme of the invention can be carried out by means of conventional techniques that comprise the separation of proteins produced by means of chromatofocussing and gel filtration of the concentrated fractions presenting greatest proteolytic activity [see Example 2]. The enzyme with purified proteolytic activity can be used for its characterisation (Example 3).

From the enzyme of the invention, it is possible to identify and isolate the DNA sequence that codes for said enzyme by means of a process comprising:
- creating gene libraries of genomic DNA (gDNA) or copy DNA (cDNA) from organisms that produce the enzyme of the invention;
- sequencing the amino terminal of the enzyme of the invention and of tryptic fragments thereof;
- designing the appropriate oligonucleotides for amplifying, by means of polymerase chain reaction (PCR), a region of the genomic clone of the organisms that produce the enzyme of the invention that serves for obtaining probes for scrutinising said gene libraries; and
- analysing and selecting the positive clones.

All these stages are described in more detail in Example 4 where obtainment of a DNA sequence that codes for the Pral protease of *T. harzianum* is shown.

The gene library of cDNA can be obtained from total RNA of the organism that produces the enzyme of the invention following a standard protocol [Chomczynski and Sacchi, 1987, Single-step method of RNA isolation by acid guanidinium thiocyanate-phenol-chlorofom extraction. Anal. Biochem. 162:156-159] with slight modifications. In a particular embodiment, the organism that produces the enzyme of the invention is *T. harzianum* CECT 2413, which is cultured in a minimum medium with fungal cell walls as the only carbon source, isolating the messenger RNA (mRNA) by means of oligo(dT)cellulose affinity chromatography. Then, the cDNA is synthesised, for example, using a commercial kit, it binds to a suitable vector and is packaged in an appropriate host.

The sequencing of the amino terminal of the enzyme of the invention and the internal fragments thereof can be carried out by any conventional process, for example, by means of the Edmans Matsudaira method [A practical guide to protein and peptide purification for microsequencing. Academic press, Inc. New York, Edmans Matsudaira (eds.) 1989].

From the information obtained from the amino terminal sequence and the internal fragments of the enzyme of the invention, a set of oligonucleotides can be designed for amplifying a specific sequence corresponding to the DNA sequence that codes for the enzyme of the invention. In a particular embodiment, the direct oligonucleotide was designed from the sequence of the amino terminal of the Pral enzyme while the inverse oligonucleotide (antisense) was designed from the sequence of the internal fragment of the Pra1 enzyme. In Example 3, the sequences of the amino terminal and an internal fragment of the Pral enzyme that served for designing the oligonucleotides used for performing the PCR are described.

The suitable fragments resulting from the PCR amplification can be labelled and used as probes to scrutinise a gene library (of gDNA or cDNA) in order to isolate the clones of interest by means of *in situ* hybridisation.

Therefore, the invention provides a DNA construct that codes for the enzyme of the invention which comprises:
a) a DNA sequence comprising the SEQ. ID. NO.: 2; or
b) a DNA sequence analogous to the sequence defined in a) which
   i) is substantially homologous to the DNA sequence defined in a); and/or
   ii) codes for a polypeptide that is substantially homologous to the protein encoded by the DNA sequence defined in a).

In the sense used in this description, the term "analogous" aims to include any DNA sequence that codes for an enzyme with proteolytic activity that has the properties i)-ii) mentioned above. Typically, the sequence of analogous DNA:
- can be isolated from any organism that produces the enzyme with proteolytic activity based on the DNA sequence shown in SEQ. ID. NO.: 2, or
- is constructed on the basis of the DNA sequence shown in SEQ. ID. NO.: 2, for example, by means of introducing conservative substitutions of nucleotides, that is, that do not give rise to another sequence of amino acids of the protease encoded by said DNA sequence shown in SEQ. ID. No.: 2, but which corresponds to the use of codons of the host organism destined to the production of the enzyme, or else by means of introducing substitutions of nucleotides that give rise to a different sequence of amino acids and, therefore, possibly to a different protein structure that could give rise to a mutant protease with properties different to those of the native enzyme. Other examples of possible modifications include the insertion of one or more nucleotides into the sequence, the addition of one or more nucleotides at either of the termini of the sequence, or the deletion of one or more nucleotides at either terminal or in the interior part of the sequence. For example, the sequence of analogous DNA can be a subsequence of the DNA sequence shown in any of the sequences shown in SEQ. ID. No.: 2.

In general, the sequence of analogous DNA is substantially homologous to the DNA sequence that codes for an enzyme with proteolytic activity of the invention, for example, SEQ. ID. No.: 2, in other words, it presents a level of homology of nucleotides of at least, 70%, preferably at least 85%, or more preferably, at least 95% with respect to any of the aforementioned sequences.

In a particular embodiment, the DNA sequence provided by this invention has the sequence of nucleotides shown in SEQ. ID. No.: 2, or a fragment thereof that codes for a protein that maintains the proteolytic activity.

In another particular embodiment, the DNA sequence provided by this invention, or fragment that encodes a protein that maintains the proteolytic activity originates from *T. harzianum* and comprises the sequence of nucleotides shown in SEQ. ID. No.: 2, or a part thereof. In a preferred embodiment, said DNA sequence is derived from *T. harzianum,* has the sequence of nucleotides shown in SEQ. ID. No.: 2 and codes for the Pral protease of *T. harzianum.*

The DNA sequence that codes for the enzyme of the invention may originate not only from *T. harzianum* but also from any other strain of *Trichoderma* or from any host organism transformed with said DNA sequence.

Alternatively, the DNA sequence that codes for the enzyme of the invention can be isolated by means of conventional techniques from the DNA of any organism, by means of the use of probes or oligonucleotides prepared from the information on the DNA sequence provided by this description, or by means of initiators (by PCR).

The DNA sequence that codes for the enzyme of the invention, or the construct containing it, can be inserted into an appropriate vector. Therefore, the invention also relates to a vector, such as an expression vector, comprising said DNA sequence, or a construct that contains it. The choice of vector will depend on the host cell in which it is to subsequently be introduced. By way of example, the vector where said DNA sequence is introduced can be a plasmid or a vector that, when it is introduced into a host cell, is integrated into the genome of said cells and replicates along with the chromosome (or chromosomes) in which it has been integrated.

In the vector provided by this invention, the DNA sequence that codes for the enzyme of the invention should be operatively connected to a promoter and to a terminating sequence. The promoter can be any DNA sequence that shows transcriptional activity in the chosen host cell and can be derived either from genes that code for the homologous or heterologous proteins of the host cells. The processes used to bind the DNA sequence that codes for the enzyme of the invention to the promoter and to the terminating sequence, respectively, and for inserting said construct into a vector are well known by those skilled in the art and have been described, for example, by Sambrook et al. [Molecular Cloning. A Laboratory Manual, Cold Spring Harbor, NY, 1989].

The invention also provides a cell that comprises a DNA sequence that codes for the enzyme of the invention, or a DNA construct that contains said sequence or said vector mentioned above. The host cells that can be transformed with the DNA sequence that codes for the enzyme of the invention can be prokaryotic cells or, preferably, eukaryotic cells, such as cells of vegetable tissue or fungal cells, for example, yeast cells or cells of filamentous fungi. The transformation of these cells may be performed by means of conventional techniques, for example, by means of techniques that implicate the formation of protoplasts and the transformation thereof followed by the regeneration of the cell wall. The transformation of cells of vegetable tissue can be very interesting from several points of view, for example, for increasing the resistance to phytopathogenic fungi.

Because the enzyme of the invention, optionally, has the capacity to irreversibly deactivate, by means of proteolysis, structural proteins or those with enzymatic activity implicated in the attack of pathogens on animals or plants and/or which determine the virulence or pathogenesis of pathogens on animals or plants, the DNA construct provided by this invention, which encodes the enzyme of the invention, can be used to reduce the virulence or pathogenesis of pathogens on animals and plants, or for increasing the resistance of animals and plants to pathogens, for example, for increasing the resistance of plants to fungi of the genus *Botrytis by* means of deactivation of the structural protein(s) or enzyme(s) implicated in the attack of the pathogen on the plant. Accordingly, in a particular embodiment, the DNA construct provided by this invention is used in obtaining transgenic plants able to express the enzyme of the invention to improve the resistance to pathogens by means of irreversible deactivation of enzymes and proteins responsible for the attack on the plant. In order to obtain these transgenic plants, it is possible to proceed with conventional antisense mRNA techniques and/or overexpression (silently in one sense), or others, for example, using binary vectors or other vectors available for the different techniques of transformation of plants available at present.

Therefore, the invention also provides a transgenic cell of a plant that comprises a DNA construct of the invention that has a promoter, functional in said plant, operatively bound to a DNA construct of the invention, or to a fragment thereof.

A transgenic plant comprising, at least, one of said transgenic cells, constitutes an additional object of this invention. In a particular embodiment, said transgenic plant is a strawberry plant.

The invention also provides a method for the production of an enzyme of the invention, that comprises culturing a suitable host cell containing the DNA sequence that codes for the enzyme of the invention in conditions that allow the production of the enzyme and recovery of the enzyme from the culture medium.

The medium used for culturing the transformed host cells can be any medium suitable for culturing the host cells in question. The peptidase expressed can be, advantageously, secreted to the culture medium and can be recovered by means of a process as described in Example 2 or by any other conventional isolation process for proteins that comprises the separation of the cells from the culture medium, the precipitation of the proteins and the separation by means of chromatographic methods.

The invention also provides an enzymatic preparation comprising at least an enzyme with proteolytic activity of the invention. This enzymatic preparation is useful for the degradation or modification of materials containing peptide bonds, that is, materials that contain proteins, peptides or amino acid moieties bound by peptide bonds, such as cell walls of prokaryotic or eukaryotic organisms, for example, fungal cell walls, as these cell walls contain chitin and/or glucan polymers embedded in, and covalently bonded to, a protein matrix, or cuticles of insects or arachnids that comprise structures based on polysaccharides covalently bound to proteins.

The enzymatic preparation provided by this invention may contain between 0.01% and 100% by weight of the enzyme with proteolytic activity of the invention.

In a particular embodiment, the enzymatic preparation provided by this invention is an enzymatic preparation of a single component and mostly contains an enzyme with proteolytic activity of the invention.

In another particular embodiment, the enzymatic preparation provided by this invention comprises multiple enzymatic activities, for example, different enzymatic activities required for the modification or degradation of microbial cell walls. By way of example, said enzymatic preparation may include lytic enzymes, in particular of microbial origin (fungal or bacterial), for example, derivatives of different species of the genera *Trichoderma, Oerskovia, Arthrobacter, Rhizotocnia, Staphylococcus* or *Streptomyces.* It can also contain one or more enzymes able to modify or degrade cell walls, for example, enzymes with cellulolytic, mananolytic, chitinolytic or proteolytic activity, such as cellulases, β-(1,6)-glucanases, β-(1,3)-glucanases, mananases, endo- o exo- chitinases, chitosanases, proteases, α- or β-manosidases, mutanases, etc. These enzymes may come from any producing organism thereof, such as different species of the genus *Aspergillus* or those mentioned above in relation with the lytic enzymes.

The enzymatic preparation of the invention can be prepared by conventional methods and can be present in liquid or solid form, for example, in the form of a granulate. The enzymatic preparation may contain additives, for example, stabilisers that prolong the stability thereof.

The enzyme with proteolytic activity provided by this invention can also have an affinity for fungal cell walls, or for cuticles of insects and arachnids, and so they may help in the degradation of fungal cell walls, for example, phytopathogenic fungi, such as *C. acutatum, B. cinerea,* etc., or in the degradation of cuticles of insects, arachnids, etc.

Therefore, the invention also provides an antifungal composition comprising at least an enzyme of the invention along with at least a fungicide, for example, a chemical fungicide. Any of those normally used can be used as a chemical fungicide, preferably, a chemical fungicide selected from the group formed by the chemical fungicides that affect the membrane, the chemical fungicides that affect the synthesis of the cell wall and mixtures thereof. Optionally, the antifungal composition of the invention may contain, in addition, at least a protein with activity of degradation of cell walls can be used, if desired, in the antifungal composition of the invention.

The antifungal composition of the invention, which may contain between 0.01% and 99.99% by weight of the enzyme of the invention, may be prepared by conventional methods and may be presented in liquid or solid form, for example, in the form of a granulate. The antifungal composition of the invention can also contain additives, for example, stabilisers in order to prolong the stability thereof.

Since the enzyme of the invention can be used to degrade or modify materials containing peptide bonds, for example, the proteins present in microbial cell walls, the enzyme of the invention can be used as a biofungicide against different nuisance organisms, for example, against phytopathogenic fungi (including the fungi that cause damage in cultures and the fungi that contaminate fruit before and after harvest), pathogenic fungi in animals (including pathogenic fungi in humans), fungi that contaminate food, surfaces and equipment, and, in general, any fungi that causes economic losses in any industrial, agricultural or livestock sector.

The enzyme of the invention can also be used for breaking or lysing the cell walls of different microorganisms to recovery products of interest produced by said microorganisms.

The enzymatic preparation of the invention can be designed at will with a composition specifically adapted for the cell wall to be broken or lysed. For example, if the cell wall to be broken contains a protein-manane complex and a glucan, the enzymatic preparation could contain, advantageously, a mixture of protease activities, mananase, chitinase and β-glucanase, in order to efficiently break said cell wall.

Other applications of the enzyme of the invention include, by way of illustration, and not limiting, the extraction of mano-proteins from the outer layer of the cell walls of yeast; the production of protoplasts from yeasts or fungi; the preparation of extracts of yeasts and fungi; improvement in the operations of filtration in processes for the production of wines, grape musts and juices; the elaboration of wines with organoleptic properties altered by overexpression of the gene in the wine yeasts; the elaboration of compositions for cleaning teeth and dentures; the elaboration of compositions for cleaning contact lenses; the elimination of fungi on coatings; treatment of tissues, for example, the removal of excessive colorant in tissues; the elaboration of compositions for removing the dental plate; the elaboration of compositions for removing biofilms deposited on surfaces, for example, on the surface of a contact lens; the manufacture of detergents for washing clothes and/or dishes, etc.

The enzyme or enzymatic preparation or the antifungal composition provided by this invention can be used in the control of nuisance organisms, for example, against phytopathogenic fungi (including the fungi that cause damage in cultures and the fungi that contaminate fruit before and after the harvest), pathogenic fungi of animals (including pathogenic fungi in humans), fungi that contaminate food, surfaces and equipment, and, in general, any fungi that causes economic losses in any industrial, agricultural or livestock sector. In the sense used in this description, the term "control" includes the reduction or paralysing of the growth and/or the germination that may result in the elimination of said nuisance organisms or in the reduction of damage caused thereby. Therefore, in a particular embodiment of this invention, said enzymatic preparations or antifungal compositions will be pharmaceutical compositions or compositions for application in the agricultural sector.

The enzyme, enzymatic preparation or antifungal composition of the invention can also be used for disinfesting, preventing and/or treating infection caused by pathogenic fungi of animals in livestock facilities, which can be infested by pathogenic fungi that grow and develop in substrates that are in contact with the animals, for example, the straw used in animal bedding, or in the stable conditions of the animals, where there is a risk that said animals are infested by such pathogens.

On the other hand, as is well known, at times, the test samples for analysis, for example, biological samples, food, etc., present contaminations due to fungi that make difficult or impede the analysis of said samples. The invention provides a solution to said problem consisting in the use of an enzyme, enzymatic preparation or antifungal composition of the invention to control the fungal contamination in said test samples for analysis by means of its application to said samples.

The antifungal composition of the invention can be used to control all types of nuisance fungi, such as those mentioned above, by means of the control mechanism known as integrated control.

The dosing of the enzymatic preparation and of the antifungal composition provided by this invention and their conditions of use can be determined on the basis of methods known in the art.

Other applications of the enzyme of the invention include its use to control pathogenesis due to insects, arachnids, etc., by means of the destruction or modification of their cuticles, as well as its use to reduce the virulence or pathogenesis of pathogens of animals or plants by means of irreversible proteolytic deactivation of structural proteins and/or enzymes implicated in the attack of said pathogens on animals and plants.

The following examples serve to illustrate the present invention and should not be considered as limiting the scope thereof.

### EXAMPLE 1

### Production of enzymes with proteolytic activity by T. harzianum CECT 2413 in liquid culture with affinity for fungal cell walls

### 1.1 Culture conditions of T. harzianum

Pre-cultures were made of *T. harzianum* CECT 2413 in 500-mL Erlenmeyer flasks containing 200 ml of minimum medium for *Trichoderma* (MM) (15 g NaH₂PO₄, 1 ml trace metals [FeSO₄·7H₂O 0.5 g, MnSO₄·H₂O 0.16 g, ZnSO₄·H₂O 0.14 g, CoCl₂ 0.37 g, distilled H₂O in sufficient quantity for (s.q.f.) 100 ml), 2% glucose as a carbon source. The pH of the medium was set to pH 5.5 with 10 M KOH and distilled water was added in s.q.f. 973.5 ml. Once the medium has been sterilised, 20 ml of (NH₄)₂SO₄ (250 mg/mL), 4.1 ml of CaCl₂ 1 M and 2.4 ml of 1 M MgSO₄ were added. The flasks were inoculated with a final concentration of 10⁶ spores/ml, and incubated at 200 rpm at 25°C for 48 hours. Then, the mycelia were collected in sterile conditions by filtration through filter paper. They were washed with an abundant amount of 2% (w/v) MgCl₂ solution and with sterile water, and immediately used to inoculate new cultures.

### 1.2 Assay of proteolytic activity in polyacrylamide gels: casein-SDS-PAGE

The detection of proteolytic activity in the culture filtrates of *T. harzianum* was performed after submitting the samples to SDS-PAGE with the substrate included in the gel. For this, the preparation of the gels was performed normally, but adding the casein substrate to the separation gel, specifically 600 µl of renaturing buffer [Tris/HCl 50 mM pH 8.0, 1% (w/v) casein (Sigma), 2 mM ethylendiamine tetracetic acid (EDTA) and 0.05% (w/v) sodium azide]. The samples to be tested were prepared in loading buffer containing sodium dodecylsulphate (SDS) as denaturing agent, but not the reducing agent 2-mercaptoethanol [Tris/HCl 0.25 M pH 6.8; 40% glycerol; 8% SDS and 0.05% bromophenol blue]. Electrophoresis was carried out at 4°C to reduce the enzymatic activity during the run. Then, the gel was incubated in renaturing buffer at room temperature while stirring for 1 hour, with two changes of buffer. This step has the aim of eliminating the SDS contained in the gel and allowing renaturing of the enzymes. Then, the gel was incubated at 37°C in sodium acetate 50 mM buffer for 6 hours to allow the proteases to act. After staining and dₑstaining the gels, the proteolytic activity is visualised as an unstained zone over a dark blue background.

### 1.3 Protein adsorption assay to fungal cell walls

The affinity on fungal cell walls of enzymes with proteolytic activity secreted by *T. harzianum* CECT 2413 was assayed following a conventional adsorption-digestion method. To do this, 2 ml aliquots of the culture filtrates of *T. harzianum*, concentrated and dialysed, were incubated with stirring at 4°C for 20 minutes with an equal volume of a suspension at 1% (w/v) of purified fungal cell walls. Then, the samples were centrifuged at 12,000 rpm in a Sorvall model RC5-C centrifuge with an SS34 rotor for 10 minutes. The collected supernatants were incubated once more with new suspensions of cell walls twice more. Finally, the cell walls were pooled and washed three times with 5 ml of potassium phosphate buffer solution 70 mM pH 6.0 supplemented with 1M NaCl. The proteins that had not adsorbed to the cell walls (which remained in the supernatants) were precipitated and dialysed. The cell walls with the proteins adsorbed were resuspended in 1 ml of sodium acetate buffer 50 mM pH 5.5 and incubated in Eppendorf tubes at 37°C for 24 hours to allow the digestion of the cell walls and the release of the adsorbed proteins. Then, the mixture was centrifuged at 13,000 g for 5 minutes, the supernatant collected and dialysed against distilled water. The analysis of the proteases present in the different fractions was carried out by means of an assay of the activity in casein gel-SDS-PAGE [Example 1.2].

### 1.4 Identification of proteins with proteolytic activity

In order to identify proteins with proteolytic activity secreted by *T. harzianum* CECT 2413, pre-cultures of the fungus were performed in minimum mineral medium (MM) supplemented with 2% glucose as indicated in Example 1.1. Once the mycelium had grown, it was collected and transferred to an MM medium containing cell walls purified from *C. acutatum* (conditions of simulated mycoparasitism) or 2% glucose (conditions of repression by carbon source for other enzymes described) as the only carbon source.

The suspensions of cell walls of *C. acutatum* and *B. cinerea* were prepared from mycelium. The cells were broken in a mortar in the presence of liquid nitrogen, and washed several times with 2M NaCl and then with distilled water, centrifuged for 5 minutes at 8,000 rpm in the Sorvall model RC5-C centrifuge with GSA rotor, and collecting the precipitate after each wash. Then, the cells obtained were collected by filtration in kitasate, frozen at -80°C and lyophilised for 12 hours in a Virtis Centry™ lyophiliser, keeping them at room temperature until use. The purification degree was determined by observing the absence of cytoplasmatic material and plasmatic membrane material with an optical microscope.

The culture filtrates of *T. harzianum* harvested after 9, 24 and 48 hours, concentrated and dialysed, were analysed by means of an assay of proteolytic activity in casein gel-SDS-PAGE [Example 1.2].

From the cultures in presence of cell walls, a single band of activity was detected at a height below that expected for the protease Prb1 of 31 kDa described in *T. harzianum* by Geremia et al (Geremia, R.A., Goldman, G.H., Jacobs, D., Ardiles, W., Vila, S.B., van Motagu, M. and Herrera-Estrella, A. (1993). Molecular characterization of the proteinase-encoding gene, prb1, related to mycoparasitism by *Trichoderma harzianum*. Molecular Microbiology 83: 603-613), and which became more intense with increased culture time. In the cultures kept for 24 or 48 hours, other zones of proteolytic activity were also observed that did not always appear to be well defined. From the culture filtrates with glucose as carbon source, no bands of activity were detected, except for a very weak band after 48 hours of culture.

Casein used as substrate in this type of assays of proteolytic activity is usually used for the detection, in principle, of all types of proteases; nonetheless in the detection of metallo-proteases the use of gelatine is recommended (Wang, K.K.W., 1999. Detection of proteolytic Enzymes using Protein substrates. In: Proteolytic Enzymes. Tools and Targest. Pages 49-62. Springer-Verlag Berlin Heidelberg, NY). Assays by means of gelatine-SDS-PAGE of the previous supernatants did not reveal new bands of activity, and those already observed disappeared or appeared with less intensity.

In order to know the affinity for fungal cell walls of the enzymes with proteolytic activity produced by *T. harzianum* CECT 2413 following the protocol described in Example 1.4, 2 ml aliquots of supernatant of culture kept for 48 hours in the presence of cell walls were submitted to an adsorption-digestion process with cell walls purified from *C. acutatum* or from *Botrytis cinerea,* as described in Example 1.3. In the fraction adsorbed at the cell walls of *C. acutatum* or of *B. cinerea a* single band of activity was detected, while the remaining isoenzymes remained exclusively in the fraction that had not adsorbed.

The affinity of said enzyme with proteolytic activity for the structure of the cell wall suggested that it could form part of the battery of enzymes implicated in mycoparasitism, and so it was purified and characterised.

### EXAMPLE 2

### Purification of an enzyme of T. harzianum with proteolytic activity with affinity to fungal cell walls

The purification of enzymes with proteolytic activity obtained from the protocol described in Example 1 was performed from the culture filtrate of *T. harzianum* CECT 2413 kept for 48 hours in a MM medium with cell walls of *C. acutatum* as the carbon source. To do this, 2 ml of filtrate dialysed and concentrated 75 times were submitted to chromatofocussing to separate proteins on the basis of their isoelectric point. The elution profiles obtained show two peaks of proteolytic activity on azocasein, one eluted at basic pH, coinciding with the main protein peak, and another at acid pH. The isoenzyme that coincided in electrophoretic mobility with the band corresponding to the protease with affinity for the cell walls of fungi and which was the largest one of interest, was localised in this second peak by means of the assay of activity in the casein gel-SDS-PAGE (Example 1.2), which indicates that this was an acid protease with Ip of 4.7-4.9.

The fractions were mixed, concentrated in Centricon-10 (Amicon) and submitted to gel filtration chromatography. The fractions that presented greatest proteolytic activity were pooled and concentrated once more in Centricon-10. The subsequent analysis by SDS-PAGE and casein-SDS-PAGE allowed the homogeneity of the preparation obtained to be checked, in which a single band of protein and proteolytic activity, respectively, were observed. The purified enzyme with proteolytic activity and acid Ip was denominated Pral and has been used for its characterisation.

### EXAMPLE 3

### Characterisation of the Pra1 protease

### 3.1 Molecular weight

The molecular weight calculated after SDS-PAGE and staining with Coomasie blue was approximately 28.5 kDa [Laemmli, E.K. (1970). Cleavage of structural proteins during the assembly of bacteriophage T4. Nature 227: 6]. When the reducing agent 2-mercaptoethanol was added to the loading buffer, no changes were observed in the molecular weight, which indicates the lack of sub-units linked by disulphide bridges, at least, of different molecular weight.

### 3.2 Microsequencing

The sequencing of peptides obtained from the purified protease Pral was performed with the double aim of comparing the sequences of amino acids with those contained in the databases and performing the design of degenerate oligonucleotides that would allow the gene that codes for Pral to be cloned.

The sequencing of the amino-terminal and of the internal peptides of Pral was performed by the sequencing service Eurosequence b.v. (Groningen, Holland). For each one of the sequences, 1 nmol of purified protein was used, and subsequently cut from the gel after SDS-PAGE and staining with Coomasie blue. Obtaining internal peptides was performed by enzymatic digestion of the protein with trypsin, and subsequent extraction and purification using RP-HPLC. The sequencing process was performed following the Edman degradation method in an automatic sequencer from Applied Biosystems model 494 connected in phase with an RP-HPLC apparatus for the identification of the released PTH-amino acids.

Table 1 shows the sequences obtained from the amino terminal and those of an internal peptide of Pral. Both showed similarity with the sequences of amino acids of proteases such as trypsins (or trypsin like) mainly, kallikrein, or plasminogen activator. All these enzymes for part of the S1 family of the serine-peptidases whose representative enzyme is chymotrypsin and which consists of enzymes with endopeptidase activity.

**Table 1**

| **Peptide sequences** | | | |
|---|---|---|---|
| **Peptides of Pral** | **Proteins** | **Organism (Kingdom*)** | **Id. (%)** |
| **Amino-terminal**: | Trypsin ALP1 | *Cochliobulus carbonum* (Fungi) | 84.6 |
| IVGGTTAALGEFP | Trypsin I | *Ascatus fluviatilis* (Metazoa) | 84.6 |
| | Trypsin precursor | *Pacifastus leniusculus* (Metazoa) | 84.6 |
| | Trypsin-type protease SNP-1 | *Phaeosphaeria nodorum* (Fungi) | *76.9* |
| | Trypsin type protease precursor | *Metarhizium anisopliae* (Fungi) | *76.9* |
| | Precursor of trypsinogen | *streptomyces fradiae* (Bacteria) | 76.9 |
| | Trypsin precursor | *streptomyces griseus* (Bacteria) | 76.9 |
| | Trypsin-type protease | *streptomyces exfoliatus* (Bacteria) | 76.9 |
| | Serine protease precursor chymotrypsin type | Haliotis *rufescens* (Metazoa) | 76.9 |
| | Serine-protease type trypsin SP-8 | *Ctenophalides felis* (Metazoa) | 76.9 |
| | Precursor of plasma kallikrein | *Mus musculus* (Metazoa) | 76.9 |
| | Precursor of trypsin | *Fusarium oxysporum* (Fungi) | 69.2 |
| **Internal:** | Precursor of trypsin | *Fusarium oxysporum* (Fungi) | 73.3 |
| DSXSGDSGGPIIDP SG | Trypsin type protease SNP-1 | *phaeosphaeria nodorum* (Fungi) | 73.3 |
| | Trypsin type protease precursor | *Metarhizium anisopliae* (Fungi) | 66.6 |
| | Serine protease (trypsin) | *Mycobacterium tuberculosis* (bacteria) | 66.6 |
| | Precursor of trypsin | *Phedom cocleariae* (Metazoa) | 66.6 |
| | Trypsin ALP1 | *Cochliohulus* carbonum (Fungi) | 60.0 |
| | Plasminogen activator | *Scolopendra subspinipes* (Metazoa) | 60.0 |
| | Trypsin type proteases (SP-2, SP-6, SP-28, SP-40) | *Ctenocephalides felis* (Metazoa) | 60.0 |
| Id: Identity | | | |

The sequences of the amino-terminal peptide and of the internal peptides of Pral are gathered in SEQ. ID. NO.: 1 which contains the complete putative sequence of amino acids of Pra1.

### 3.3 Determination of the type of peptidase by means of specificity assays for substrates and inhibition of the proteolytic activity

### A. Assay of the proteolytic activity in liquid phase.

The proteolytic activity of the enzyme can be assayed using azocasein as substrate. The reaction mixture consists of 250 µl of sodium acetate buffer 100 mM pH 5.5 which contains the sample of enzyme, 125 µl of 0.1% Brij 35 and 125 µl of azocasein (Sigma). The reaction mixture is incubated at 30°C for a period of time comprised between 1 and 3.5 hours. The reaction is stopped by adding 200 µl of 10% TCA (trichloracetic acid), and the supernatant is measured at 366 nm. A unit of activity represents the hydrolysis of 1 µg of azocasein per minute in the assay conditions.

### B. Specificity for substrates

The differences in specificity for the substrate are, in general, more useful in the characterisation and classification of exopeptidases. Nonetheless, the preferential catalytic activity for certain residues of amino acids of many endopeptidases is known (Powers Powers, J.C. and Kam, C. (1995) Peptide Thioster substrates for serine peptidases and metalloendopeptidases. In: Methods in Enzymology, 248. 3-18), and thus, by assays of activity on certain synthetic peptides, the nature thereof can be analysed.

Taking into account the information obtained in Example 3.2, the endopeptidase activity of Pral on 3 synthetic peptides (Sigma) is assayed with the amino and carboxyl terminals blocked, with allows the different types of activities to be differentiated within the S1 family of peptidases:
N-acetyl-Ile-Glu-Ala-Arg-pNA (Arg-pNA) for trypsins,
N-succinyl-Ala-Ala-Pro-Leu-pNA (Leu-pNA) for elastases, and
N-succinyl-Ala-Ala-Pro-Phe-pNA (Phe-pNA) for chymotrypsins/ subtilisins (this latter belongs to the S8 family).

The enzymatic solution (75 ng) was prepared in 90 µl of 100 mM sodium phosphate buffer and preincubated for 5 minutes at 30°C. The reactions were initiated by addition of 10 µl of preheated 10mM substrate solution (prepared from 100 mM solutions in DMSO stored at -20°C). After 20 minutes of incubation, the reactions were stopped by addition of 50 µl of 2% (v/v) acetic acid. Then, the absorbance of 100 µl at 405 nm was measured in microtitre plates with 96 wells (Inmunoplate Maxisorp, NUNC) using a Titertek Multiskan Plus 311 A0 (Flow Laboratories) automatic reader, and the nmoles of p-nitroaniline (p-NA) released per minute were calculated. In parallel, enzyme blanks with no substrate and substrate without enzyme were also processed. The standard calibration line was constructed with solutions of para-nitroanaline (p-NA) (Sigma) at known concentrations (0-1.25 mM) prepared from an initial 5 mM solution. This solution was prepared by dissolving p-NA beforehand in a minimum volume of ethanol.

The lytic activity of Pral on the peptide Arg-pNA confirmed its endopeptidase identity, and in addition, showed the preference of this protease for an apolar residue (Arg) at the P₁ position, characteristic of trypsin type enzymes. The specific activity of Pral on this substrate was 94,800 U/mg in optimum conditions of activity (30°C and pH 7.5). Values for Kₘ, K_{cat}, and K_{cat}/Kₘ, of 0.22 mM, 39.64 s⁻¹ and 180.18 mM⁻¹s⁻¹ were calculated, respectively.

The determination of the kinetic parameters Kₘ, K_{cat}, and K_{cat}/Kₘ was performed from the initial rates calculated in assays with different concentrations of substrate (0.05-2 mM). For this, the substrate was prepared in 0.99 ml of 100 mM sodium phosphate buffer pH 7.5 and preincubated at 30°C. Then the reaction was started by the addition of 10 µl of the purified proteases Pral (2.5 ppm) preheated to the same temperature. The activity was monitored for 10 minutes in a Shimadzu UV-160A spectrophotometer at 405 nm kept at 30°C by a thermostat. A unit of activity represented the release of one 1 nmol of p-NA per minute in the assay conditions. Solutions of enzymes without substrate, and substrate without enzyme were prepared as blanks. The standard calibration curve was constructed with solutions of p-NA (Sigma) at known concentrations (0-200 µM), prepared as indicated above. The kinetic parameters were calculated from representations of double reciprocals or by Lineweaver-Burk (1/V vs 1/S).

No lytic activity of Pral on Leu-pNA or Phe-pNA was observed, which indicates the absence of elastase and chymotrypsin/subtilisin activity, respectively.

### C. Inhibition of proteolytic activity

In order to determine the catalytic type to which Pral belongs, specific inhibitors were used of the different known catalytic mechanisms. The effect of said inhibitors on the activity of Pral was analysed by preincubating the enzymatic solution for 30 minutes at 30°C in 100 mM phosphate buffer pH 7.5 with said specific inhibitors of different known catalytic mechanisms:
- 1 mM PMSF (100 mM initial solution in isopropanol),
- 1 mM EDTA (100 mM initial solution in water),
- 0.1 mM Pepstatin (10 mM initial solution in DMSO),
- 1 mM Iodoacetamide (100 mM initial solution in water, prepared at the time of use).

In parallel to the enzymatic assay, controls were also performed in the presence of organic solvent in which the inhibitor was dissolved. The residual activity was determined by the percentage of activity in absence of inhibitor.

As might be expected for a serine-peptidase, the lytic activity of Pral was drastically inhibited in the presence of PMSF. This irreversible inhibitor can also act on cysteine-peptidases, however, the alkylating agent, iodoacetamide, specific inhibitor of these, showed a weak effect on the activity of Pral. Using inhibitors such as pepstatin or EDTA, Pral also conserved more than 90% of its activity.

The results obtained allow the affirmation to be made that Pral is a serine-endopeptidase (EC 3.4.21). It was confirmed that Pral belongs to the S1 family, and particularly, to the group of trypsin type enzymes (EC 3.4.21.4) by analysing the complete amino acid sequence of the protein.

### 3.4 Effect of temperature on the activity and stability of Pra1

In order to determine the optimum temperature for activity of Pra1, the enzymatic assay was performed over a range of temperatures comprised between 30°C and 85°C. The effect of temperature on the stability of Pra1 was determined by preincubating the enzymatic solution in 100 mM sodium phosphate buffer pH 7.5 for 20 minutes at different temperatures (30-85°C). The substrate used in these assays was the synthetic peptide N-acetyl-Ile-Glu-Ala-Arg-pNA, specific for trypsins.

The effect of temperature on the activity of Pral proteases showed that its optimum temperature is close to 35°C. At 45°C, the activity dropped drastically to 16%, and above 55°C, no activity was detected. Given that at 30°C, Pral maintains 95% of its activity, and this temperature is close to the conditions in which *Trichoderma* is cultured, it was taken as the normal temperature for the assays of activity.

### 3.5 Effect of pH on the activity and stability of Pra1

For the determination of the optimum pH for activity of Pral, the assay was carried out in the following buffer systems: 100 mM sodium acetate (pH 3-5.5), 100 mM sodium phosphate (pH 6-7), and 100 mM Tris/HCl (pH 7.5-9). The effect of pH on the stability of Pral was determined by pre-incubating the enzymatic solution (750 ng) for 24 hours at 4°C in 10 µl of the aforementioned buffers. Then, aliquots of 10 µl were taken for performing the assay in standard conditions.

The obtained results show that the optimum pH for activity of Pral is comprised between 7.0 and 8.0. The activity decreased rapidly when the assay was performed in buffers with pH greater than 8.5 or less than 6.5.

The assay of activity of Pral performed in optimum conditions (30°C and pH 7.5) after preincubating the enzyme for 24 hours in buffers at different pH showed that Pra1 is more stable at acid pH. The activity decreased as the pH of preincubation increased, with a reduction of 40% at pH 7. No proteolytic activity was detected after preincubation at pH 9.

### EXAMPLE 4

### Cloning of a sequence of cDNA that codes for Pra1

The cloning of the gene that codes for the protease Pral of *T. harzianum* was performed from a gene library of cDNA representative of the population of mRNA molecules that are transcribed in *T. harzianum* CERT 2413 when it is cultured in conditions of simulated mycoparasitism (for 9 hours in the presence of fungal cell walls as the only source of carbon).

4.1 Obtaining total RNA of *T. harzianum* The extraction of RNA is performed from 50 mg of mycelium of *Trichoderma* ground up in a mortar in the presence of liquid nitrogen. The mycelium is transferred to 10 ml tubes, to which 4 ml of "ARNol yellow" lysis solution are added [20 ml of solution D (4 M guanidinium isothiocyanate, 25mM sodium citrate pH 7,0, 0.5% (w/v) sarcosyl, autoclave and add 2-mercaptoethanol at a final concentration of 0.7% (v/v)], 2 ml of 2 M sodium acetate and 20 ml of phenol acid]. The samples were homogenised by stirring in vortex and mixing with a micropipette. They were then incubated at room temperature for 5 minutes and transferred to Eppendorf tubes in aliquots of 1 ml. 0.2 ml of chloroform were added to each tube, stirring in vortex for 15 seconds, and incubating at room temperature for 2-3 minutes. They were then centrifuged at 12,000 g and 4°C for 15 minutes. One volume of isopropyl alcohol was added to the supernatant collected, with subsequent incubation at room temperature for 15 minutes to allow the precipitation of RNA. Then the RNA was collected by centrifuging at 12,000 g and 4°C for 10 minutes and washed with 1 ml of 70% ethanol stirring in vortex until the precipitate separates from the tube. It was centrifuged at 4,000 g and 4°C for 5 minutes. Then the ethanol was eliminated by inversion, and the precipitate allowed to dry in air and resuspended in 40 µl of water. The RNA obtained was quantified by spectrophotometry and its integrity checked by 1% agarose gel electrophoresis. It was stored at -80°C.

From 1 mg of total RNA extracted from the mycelium grown in the aforementioned conditions, 7.8 µg of mRNA were purified by affinity chromatography. The mRNA was used for assembling the gene library in the vector Uni-ZAP® XR (Stratagene).

### 4.2 Assembly and manipulation of a gene library of cDNA of T. harzianum CECT 2413 in the vector Uni-ZAP® XR

The cloning system ZAP-cDNA® Gigapack® III Gold Cloning Kit designed by Stratagene (Ref. 200450) was used. The experimental procedure was performed mainly according to the instructions of the protocol provided by the commercial firm both for the assembly and for the manipulation of the gene library.

From the mRNA purified from 1 mg of total RNA, the processes of synthesis of the first and second chain of the cDNA were carried out, the filling of the terminals of the double-strand cDNA, the assembly thereof on the EcoRI adaptors, the phosphorylation of the terminals, and the digestion with the restriction endonuclease xhoI, using the reagents provided by the commercial company Stratagene, and following in detail the recommendations of the protocol attached, with the exception that the radioactive nucleotides were not incorporated for monitoring the process. The subsequent separation of the cDNA from the excess of adaptors was performed by gel filtration chromatography using columns provided by the supplier Pharmacia. Then 150 ng, approximately, of cDNA were bound to 1 µg of DNA vector (pre-digested with the restriction enzymes *Eco*RI/*Xho*I) in a recommended reaction volume of 5 µl, of which 2 µl were used for the final process of packaging of the DNA inside the phage particles following the indications of the supplier. The efficiency of the packaging was 4,9.10⁶ recombinant phages/µg of vector, and the titre of the primary gene library was 4.10⁶ plate forming units (pfU)/ml (2.10⁶ en total), with only 0.3% of phages being non-recombinant. The primary gene library, resulting from the process described, was stored at 4°C for a time of less than 1 month before amplification.

Given the instability of the primary gene libraries, it is necessary to amplify them despite the loss of representativity of the least abundant clones. The process was performed by infecting 600 µl of *E. coli* XL1-Blue cells with volumes corresponding to 50,000 pfu (plate forming units) of the primary gene library. The infection mixture was incubated at 37°C for 15 minutes to allow attack of the phages and then it was added to the tubes with 7 ml of NZY (bacterial culture medium appropriate for the growth of phages) with preheated covering at 48°C. The tubes were stirred in vortex and the content immediately spread on 15 cm Petri dishes with solid NZY medium. The plates were incubated at 37°C until confluent lysis halos were observed. Then 10 ml of SM buffer [100 mM NaCl, 10 mM MgSO₄, 50 mM Tris-HCl, pH 7.5, 0.01% gelatine (w/v)] were added and it was incubated once more at 4°C in a gentle rocking motion to allow diffusion of the bacteriophages to the buffer. After 12 hours, it was collected and the buffer of the different plates was mixed, washing each one of them with 2 ml of additional buffer. The mixture collected was shaken vigorously in the presence of 5% (v/v) chloroform, and incubated for 15 minutes and room temperature. After centrifuging at 500 g for 10 minutes, the supernatant with the bacteriophages free of cell remains was transferred to a glass recipient. At this moment, the cDNA gene library was ready to be used. It was titred and stored at 4°C in the presence of 0.3% (v/v) chloroform, and also at -80°C in the presence of 7% DMSO. The amplification of 1 million pfu provided a gene library amplified 2.5x10⁶ times, with a titre of 1.6.10¹⁰ pfu/ml.

### 4.3 Searching the gene library

### A. Obtaining the probe of pra1

The search for the cDNA clone corresponding to the Pral protease in the amplified gene library was performed by hybridisation using a DNA probe obtained beforehand by PCR from the gene library of phagemids pBluescript SK(-) derived from the bulk scission of 20.10⁶ pfu. Said gene library of phagemids pBluescript SK(-) had a titre of 3.3.10⁶ phagemids/ml (66.10⁶ in total).

In order to obtain said probe, a PCR was performed from the phagemid DNA extracted from a million bacterial clones, using the degenerate oligonucleotides as initiators: 
PPra1:    5'ACTGCIGC(G/T)TTIGGIGA(A/G)TT(T/C)CC-3' (sense)
          [SEQ. ID. NO.: 3]; and
 and 
Pra1IA:    5'-GGGTCTAT(T/G)ATIGGICCICC-3' (antisense) [SEQ.
           ID. NO.: 4]
 designed from the sequence of amino acids of the amino-terminal peptides and internal peptides, respectively, of the protease Pral. The reaction mixture was prepared in a final volume of 25 µl containing buffer of PCR 1X, prepared from 10X buffer, provided by the enzyme, 1.5 mM MgCl₂, dNTPs 200 µM, 1.25 units of *Taq* polimerase (Eco*taq*), 4 µM of each initiator oligonucleotide, and approximately 10 ng of phagemid DNA. The amplification conditions consisted of an initial denaturing cycle at 95°C for 3 minutes, followed by 35 cycles at 95°C (denaturing) for 1 minute, 55°C (hybridisation of the initiators) for 1 minute and 72°C (extension) for 1 minute, finishing with a final extension cycle at 72°C for 5 minutes. The amplified products were analysed by electrophoresis in 1.2% agarose gel and the fragments of interest were extracted from the gel and subcloned in *E. coli* using the plasmid pGEM® -T as a vector, for subsequent sequencing.

As a result of the reaction, an amplification product was repeatedly observed of approximately 550 pb which, once recovered from the gel, was subcloned into a vector pGEM® -T (Promega). The sequencing of this fragment made it possible to check that it contained the sequences that encoded for the known peptides of Pral, such that it is used as a probe to examine the gene library in the vector Uni-ZAP® XR and isolate the complete cDNA of *pra1.*

### B. Transfer of bacteriophage DNA to membranes

Petri dishes 15 cm in diameter were prepared with approximately 50,000 pfu from the gene library of amplified cDNA. Once the lysis halos had been obtained, the plates were allowed to cool so that the covering agar acquired greater thickness. A nylon membrane (Hybond-N, Amersham) was placed over the agar surface avoiding bubbles. The membrane was left for 5 minutes to allow the transfer of phages, and it was marked asymmetrically with a needle to allow orientation when it came to recovering the clone or clones of interest from the plate. The membranes were carefully withdrawn from the plates (which were stored at 4°C) and treated with Southern I denaturing solution for 2 minutes, followed by Southern II neutralisation solution for 5 minutes, and finally with SSC 2X buffer, prepared from SSC 20X, for 5 minutes. These treatments were performed by depositing the membranes, with the side that was in contact with the phages upwards, over Whatman 3MM paper soaked in the corresponding solution. Then, the membranes were allowed to dry in air and the transferred DNA was covalently fixed to the membrane. Then, the membranes were incubated at 65°C with constant stirring in hybridisation solution [SSPE 5X (SSPE 20X: 3.6 M NaCl, 0.2 M NaH₂PO₄, 20 mM EDTA pH 7.7), Denhardt Solution 5X (Denhardt Solution 100X: 2% BSA (w/v), 2% Ficoll™ (w/v), 2% PVP (w/v)), 0.5% SDS (w/v)] for 2 hours. Then, the radioactively labelled DNA probe was added and the incubation continued for 12-18 hours. After this time, the probe was removed and the membrane washed with a wash solution [SSPE 2X, 1% SDS (w/v)] at room temperature for 15 minutes, and then twice more at 65°C. Finally, the membranes were wrapped in a transparent plastic film to prevent them from drying, and exposed with amplifying screens for the detection of positive clones.

Of a total of 2x10⁵ clones scrutinised, 3 bacteriophages were identified that showed positive hybridisation signal. These were isolated in two successive hybridisation rounds. Once they had been recovered as pBluescript SK(-) phagemids, the inserts of each one were released by digestion with the enzymes *EcoR*I/*Xho*I. The three clones showed an insert size close to 1 kb. From the sequence obtained from one of these clones (pSKPral) the sequence of amino acids deduced was obtained. This sequence of amino acids contained the peptides (amino terminal and internal) sequenced from the purified Pra1 protease, confirming that cloned cDNA was the one that encoded for this protein.

### 4.4 Analysis of the sequence

The cloned cDNA presented a size of 954 pb, including the tail of polyA of the 3' terminal, with a open reading pattern of 777 pb that encodes a protein of 258 amino acids with a theoretical molecular weight of 25,784 Da. This deduced sequence of amino acids contains the peptides (amino-terminal and internal) sequenced from the purified Pral protease, confirming that cloned cDNA is the one that codes for this protein.

The known sequence of the amino-terminal peptide of Pral indicates that the mature protein starts at residue I¹, which points to the deduction that Pral is synthesised as a precursor with an extension at the amino-terminal end of 29 amino acids. The molecular weight calculated from the sequence of the mature protein (I¹-G²²⁸) is 25,023 Da, slightly less than that determined by SDS-PAGE from purified Pral protein (this divergence between the theoretical weight and the determined weight seems to be due to possible post-translational changes, such as glycosydations).

On the other hand, the isoelectric point estimated from the sequence of mature protein is 4.91, coinciding with that observed by preparative isoelectrofocussing (4.7-4.9).

On analysing the sequence of the amino-terminal region by means of the SignalP V1.1 program, a cut-off point was determined between the amino acids G⁻¹⁰-A⁻⁹, suggesting the existence of a peptide signal constituted from the first 20 amino acids, characteristic of secreted proteins. The 9 amino acids existing between the peptide signal and the mature protein would correspond to the pro-peptide characteristic of many proteases, which in the case of the serine-peptidases of the S1 family varies from 6-9 amino acids, and which, once processed, give rise to a new aminoacid terminal that generally starts with a hydrophobic residue such as valine, methionine, leucine or isoleucine, which is in agreement with the isoleucine¹ residue presented by Pra1.

Comparison of the complete sequence of amino acids of Pral with those contained in the data banks (EMLB and Swiss Prot) showed the high degree of similarity of this protein with members of the S1 family of serine-peptidases such as trypsins and trypsin type proteins of rat, pig, etc. The maximum homology (75-80%) with identities equal to or less than 50% was presented with trypsin type proteins of filamentous fungi *(Metharrizium, Fusarium).* The sequences of fungal proteases with which they were compared were as follows:

| **Fungi** | **EMBL/Swiss Prot identification number** |
|---|---|
| *Cochliobolus carbonum* | Q00344 |
| *Fusarium oxysporum* | P35049 |
| *Metharrizium anisopliae* | Q9Y7A9 |
| *Metharrizium anisopliae* | Q9Y842 |
| *Phaeosphaeria nodurum* | 074696 |

The alignment with these sequences allowed the catalytic triad (H-D-S) characteristic of the S1 family to be recognised as well as the different families that form part of the PA clan, and which were putatively assigned to the H⁷⁰ D¹¹⁸ and S²¹³ residues of the sequence deduced from Pra1.

## Claims

1. An enzyme with proteolytic activity **characterised in that** it has a sequence of amino acids selected from:
a) a sequence of amino acids comprising the sequence of amino acids shown in SEQ. ID. NO.: 1, and
b) a sequence of amino acids substantially homologous and functionally equivalent to the sequence of amino acids shown in SEQ. ID. NO.: 1.

2. An enzyme according to claim 1, **characterised in that** it has the sequence of amino acids shown in SEQ. ID. NO.: 1.

3. An enzyme according to claim 2, **characterised in that** it has an apparent molecular weight determined in denaturing conditions of approximately 28.5 kDa.

4. An enzyme according to claim 2, **characterised in that** it has an apparent isoelectric point comprised between 4.7 and 4.9.

5. An enzyme according to claim 2, **characterised in that** it has an optimum temperature comprised between 35°C and 40°C.

6. An enzyme according to claim 2, **characterised in that** it is a serine-peptidase.

7. An enzyme according to claim 2, **characterised in that** it is a trypsin type endopeptidase.

8. A DNA construct comprising a DNA sequence that codes for an enzyme according to any of claims 1 to 7, or a fragment thereof.

9. A DNA construct according to claim 8, **characterised in that** it has a sequence of nucleotides selected from:
a) a DNA sequence comprising SEQ. ID. NO.: 2; or
b) a DNA sequence analogous to the sequence defined in a) which (i) is substantially homologous to the DNA sequence defined in a); and/or (ii) codes for a polypeptide that is substantially homologous to the protein encoded for by the DNA sequence defined in a).

10. A recombinant vector **characterised in that** it contains a DNA construct according to any of claims 8 or 9.

11. A cell **characterised in that** it contains a DNA construct according to either of claims 8 or 9, or a vector according to claim 10.

12. A transgenic cell of a plant comprising a DNA construct that has a promoter, functional in said plant, operatively linked to a DNA construct according to either of claims 8 or 9.

13. A transgenic plant comprising at least a transgenic cell according to claim 12.

14. A transgenic plant according to claim 13, that expresses an enzyme with proteolytic activity according to any of claims 1 to 7, to improve the resistance to pathogens through irreversible deactivation of enzymes and proteins responsible for attack on the plant.

15. A method for the production of an enzyme according to any of claims 1 to 7, comprising culturing a cell according to claim 11 under conditions that allow the production of the enzyme and recovery of the enzyme from the culture medium.

16. An enzymatic preparation comprising at least an enzyme according to any of claims 1 to 7.

17. An enzymatic preparation according to claim 16, comprising between 0.01% and 100% by weight of an enzyme according to any of claims 1 to 7.

18. An enzymatic preparation according to claim 16, that contains as a single component an enzyme according to any of claims 1 to 7.

19. An enzymatic preparation according to claim 16, that contains more than one enzyme according to any of claims 1 to 7.

20. An enzymatic preparation according to claim 16, that comprises, in addition, at least one enzyme selected from the group formed from cellulases, glucanases, mananases, chitinases, proteases and/or chitosanases.

21. An antifungal composition that comprises, at least, an enzyme according to any of claims 1 to 7 along with, at least, a chemical fungicide.

22. A composition according to claim 21, in which said chemical fungicide is selected from the group formed from a chemical fungicide that affects the membrane, a chemical fungicide that affects the synthesis of the cell wall, and mixtures thereof.

23. A composition according to claim 21, comprising in addition at least one protein with degradation enzymatic activity of the microbial cell wall.

24. Use of an enzyme according to any of claims 1 to 7, or of an enzymatic preparation according to any of claims 16 to 20, or of an antifungal composition according to any of claims 21 to 23, for degrading or modifying materials containing peptide bonds.

25. Use according to claim 24, in which said material that contains peptide bonds comprises structures comprising proteins or peptides.

26. Use according to claim 25, in which said structures comprising proteins or peptides are selected from fungal cell walls and cuticles of insects or arachnids.

27. Use according to claim 24, in which said enzyme, enzymatic preparation or antifungal composition is used for the preparation of protoplasts.

28. Use according to claim 24, in which said enzyme, enzymatic preparation or antifungal composition is used for the preparation of yeast extracts.

29. Use according to claim 24, in which said enzyme, enzymatic preparation or antifungal composition is used in the extraction of manoproteins.

30. Use according to claim 24, in which said enzyme, enzymatic preparation or antifungal composition is used in the production of wines, musts and juices.

31. Use according to claim 24, in which said enzyme, enzymatic preparation or antifungal composition is used in the elaboration of compositions for dental plaque removal.

32. Use according to claim 24, in which said enzyme, enzymatic preparation or antifungal composition is used in the elaboration of compositions for the cleaning of teeth and dentures.

33. Use according to claim 24, in which said enzyme, enzymatic preparation or antifungal composition is used in the elaboration of compositions for removing biofilms deposited on surfaces.

34. Use according to claim 24, in which said enzyme, enzymatic preparation or antifungal composition is used in the elaboration of compositions for the cleaning of contact lenses.

35. Use according to claim 24, in which said enzyme, enzymatic preparation or antifungal composition is used in the elimination of fungi on coatings.

36. Use according to claim 24, in which said enzyme, enzymatic preparation or antifungal composition is used for treating and/or cleaning tissues.

37. Use according to claim 24, in which said enzyme, enzymatic preparation or antifungal composition is used in the control of pathogenic organisms of plants, animals, including man and contaminants of harvests or food.

38. Use according to claim 37, in which the control of pathogenic organisms of plants, animals, including man and contaminants of harvests or food by said enzyme, enzymatic preparation or antifungal composition, is carried out by means of irreversible proteolytic deactivation of enzymes and/or structural proteins used by pathogens in their attack on plants and animals.

39. Use according to claim 24, in which said enzyme, enzymatic preparation or antifungal composition is used for disinfecting, preventing and/or treating the infection caused by pathogenic fungi of animals in farming facilities.

40. Use according to claim 24, in which said enzyme, enzymatic preparation or antifungal composition is used for controlling the fungal contamination in a test sample for analysis.


SEQUENCE LISTING

<110> NEWBIOTECHNIC, S.A.
      UNIVERSIDAD DE SEVILLA
      UNIVERSIDAD DE SALAMANCA

<120> ENZYME WITH PROTEOLYTIC ACTIVITY

<130> T HARZIANUM PROTEASE

<140>
<141>

<160> 4

<170> PatentIn Ver. 2.1

<210> 1
<211> 258
<212> PRT
<213> Trichoderma harzianum

<400> 1
Met Ala Pro Val Leu Ala Ile Ala Ser Val Leu Ala Ala Leu Pro Ala
  1               5                  10                  15

Leu Thr Met Gly Ala Ala Ile Thr Pro Arg Gly Ser Asp Ile Val Gly
             20                  25                  30

Gly Thr Thr Ala Ala Leu Gly Glu Phe Pro Tyr Ile Val Ser Leu Ser
         35                  40                  45

Thr Gly Gly Ser His Phe Cys Gly Gly Val Leu Ile Asp Ser Arg Thr
     50                  55                  60

Val Val Thr Ala Gly His Cys Thr Ile Asp Gln Arg Ala Ser Ser Val
 65                  70                  75                  80

Lys Val Arg Ala Gly Thr Leu Thr Trp Ala Ser Gly Gly Thr Gln Val
                 85                  90                  95

Gly Val Ser Ser Leu Thr Leu His Pro Ser Tyr Thr Val Asp Ser Gln
            100                 105                 110

Gly Val Pro Asp Asn Asp Val Gly Val Trp His Leu Ala Thr Ala Ile
        115                 120                 125

Pro Thr Ser Ser Thr Ile Gly Tyr Ala Thr Leu Pro Ala Ser Gly Ser
    130                 135                 140

Asp Pro Ala Ala Gly Thr Thr Leu Thr Val Ala Gly Trp Gly Thr Thr
145                 150                 155                 160

Ser Glu Asn Ser Asn Ser Leu Pro Ser Thr Leu Arg Lys Val Ser Val
                165                 170                 175

Pro Val Val Ala Arg Ala Thr Cys Asp Ser Asp Tyr Asp Gly Glu Ile
            180                 185                 190

Ser Asn Asn Met Phe Cys Ala Ala Val Ala Ala Gly Gly Lys Asp Ser
        195                 200                 205

Cys Ser Gly Asp Ser Gly Gly Pro Ile Ile Asp Pro Ser Gly Thr Leu
    210                 215                 220

Val Gly Val Val Ser Trp Gly Gln Gly Cys Ala Glu Arg Gly Phe Pro
225                 230                 235                 240

Gly Val Tyr Thr Arg Leu Gly Asn Tyr Val Ser Phe Ile Asn Ser Asn
                245                 250                 255

Arg Gly

<210> 2
<211> 777
<212> DNA
<213> Trichoderma harzianum

<400> 2
atggctcccg ttctcgccat cgcctccgtg cttgcagcac ttcccgctct caccatggga 60
gctgccatca ctcctcgtgg cagtgatatc gtcggaggaa ccactgctgc cctcggcgag 120
ttcccctaca ttgtctctct gtccactggt ggttcgcact tctgcggtgg tgttctgatc 180
gactcccgca ccgttgtcac cgctggccac tgcaccattg accagagggc ctcctctgtc 240
aaggtccgcg ctggaactct tacctgggct tccggtggca cccaggttgg tgtttcatct 300
ctgacccttc accccagcta caccgtcgat agccagggtg ttcccgacaa cgatgttggt 360
gtttggcact tggccactgc cattcctacc agctctacca tcggttatgc tactcttcct 420
gcttctggct cagaccctgc tgccggtacc accctcaccg tcgctggctg gggaactact 480
tctgagaact ccaactctct cccctccacc ctgaggaagg tttccgtccc cgtcgttgcc 540
cgcgccactt gcgacagcga ctacgatggc gagatcagca acaacatgtt ctgcgctgct 600
gttgccgccg gtggcaagga ctcctgctct ggagactctg gtggccccat cattgacccc 660
agcggaaccc tggttggtgt tgtttcttgg ggtcagggat gcgctgaccg tggattcccc 720
ggtgtttaca ctcgcctggg caactacgtc agcttcatca acagcaaccg tggttaa 777

<210> 3
<211> 23
<212> DNA

<400> 3
actgcigcdt tiggigartt ycc 23

<210> 4
<211> 20
<212> DNA

<400> 4
gggtctatda tiggiccicc 20